# EUROPÄISCHE PATENTSCHRIFT

(11) **EP 1 417 936 B1**
(45) Veröffentlichungstag und Bekanntmachung des Hinweises auf die Patenterteilung: **04.05.2011**
(21) Anmeldenummer: 03026005.3
(22) Anmeldetag: 11.11.2003
(51) Int. Cl.: A61B 18/14

(54) **Sonde mit Metallelektrode und Verfahren zur Herstellung einer solchen Elektrode**
Probe with a metal electrode and method of manufacturing such an electrode
Sonde avec une électrode métallique et procédé de fabrication d'une telle électrode

(30) Priorität: 11.11.2002 DE 10252325; 06.12.2002 DE 10257146
(43) Veröffentlichungstag der Anmeldung: 12.05.2004
(73) Patentinhaber: Admedes Schuessler GmbH, 75179 Pforzheim (DE)
(72) Erfinder: Müller, Wolfgang, Prof. Dr.-Ing. habil., 78532 Tuttlingen (DE); Flaxmeier, Erik, 76307 Karlsfeld (DE); Steiner, Ralf, 75181 Pforzheim (DE)
(74) Vertreter: Müller-Boré & Partner Patentanwälte

(56) Entgegenhaltungen:
- WO-A-94/21168
- WO-A-99/44524
- US-A- 5 471 982
- US-A- 5 681 280
- US-A- 5 725 525
- US-B1- 6 319 251

## Beschreibung

Die vorliegende Erfindung betrifft eine endoluminale Sonde zur Hochfrequenz-Thermoablation, auch Hochfrequenz induzierte Thermotherapie genannt, bei der Biogewebe durch Wärmeerzeugung mittels hochfrequenter elektrischer Felder und Ströme thermisch denaturiert werden. Die Sonde, die insbesondere auch in Kombination mit flexibler Endoskopietechnik eingesetzt werden kann, dient zur minimalinvasiven thermischen Beeinflussung von erkrankten Strukturen in Hohlorganen (z. B. Darm, Bronchien, Trachea, Ösophagus) oder in größeren Venen oder Arterien durch Hochfrequenzenergie im Frequenzbereich von 300 kHz bis 1 mHz.

US-B1-6 319 251 offenbart eine medizinische Vorrichtung und Verfahren zum Behandeln intravaskulärer Restenose mit einer erweiterbaren Drahtbaugruppenanordnung mit einer Vielzahl von vorgeformten expandierbaren metallischen Korbelementen an dem distalen Ende eines inneren Katheters zum Anwenden eines Hochfrequenzstroms an den Geweben für therapeutische Zwecke über einen Drahtführungsschaft.

WO 99/44524 offenbart eine erweiterbare PMR Vorrichtung und ein entsprechendes Verfahren mit einem Korb, der aus flexiblen Armen gebildet ist und über deren Länge Schneidelektroden trägt.

US-A-5 681 280 offenbart ein Kathetersteuersystem mit einer erweiterten Elektrodenbaugruppe an dem distalen Ende, die flexible und elastische Endsegmente aufweist, die in einer geraden Form konfiguriert sind und beim Ausüben einer Biegekraft eine gekrümmte Form annehmen.

US-A-5 471 982 beschreibt Herzaufnahme und -ablationssysteme mit einem Katheter zum Einführen in das Herz. Die Aufnahmebaugruppe hat eine Korbform mit flexiblen Elektrodenstützen, die voneinander beabstandete Fühlerelektroden tragen.

Die Aufgabe der Erfindung besteht in der Schaffung einer Sonde zur Hochfrequenz-Thermoablation, die selbst bei anatomisch variabler biologischer Umgebung einen optimierten elektrisch leitfähigen Elektrodenkontakt herstellen kann, sowie in der Schaffung eines Herstellungsverfahrens für eine derartige Sonde.

Diese Aufgabe wird durch eine Sonde zur Hochfrequenz-Thermoablation mit den Merkmalen nach Anspruch 1 sowie durch ein Herstellungsverfahren mit den Merkmalen nach Anspruch 7 gelöst. Vorteilhafte Weiterbildungen der Erfindung sind in den abhängigen Ansprüchen definiert.

Die Sonde weist einen isolierten Schlauch auf, an dessen distalem Ende eine Metallelektrode vorgesehen ist, deren räumliche Erstreckung von einer zylindrischen Gestalt zu einer sternförmigen Gestalt veränderbar ist. Durch die zylindrische Gestalt der Metallelektrode ist eine kleinlumige Navigation der Sonde innerhalb eines biologischen Lumens oder über den Instrumentierkanal eines Endoskops möglich. Nach Positionierung der Elektrode im Zielgebiet kann anschließend eine Elektrodenentfaltung zu der sternförmigen Gestalt erfolgen. Insbesondere durch die sternförmig ausgebildete Elektrode werden hier besonders gute Wirkungen bei der Thermo-Ablation erzielt, wobei ein zusätzlicher Vorteil der veränderliche Durchmesser der sternförmigen Elektrode ist. Hierdurch kann intraluminal ein lokal definierter und elektrisch leitfähiger Elektrodenkontakt trotz anatomisch variabler biologischer Umgebung hergestellt werden.

Die Metallelektrode ist durch mehrere flexible Arme gebildet, deren distale und proximale Enden miteinander verbunden sind, wobei die distalen und/oder die proximalen Enden der Arme elektrisch mit einem Anschluss zur Zuführung eines Hochfrequenzstromes verbunden sind. Somit bilden die flexiblen Arme selbst die Elektrode, d. h. es sind keine zusätzlichen Punktelektroden oder dergleichen erforderlich. Hierdurch ist einerseits die Herstellung der Sonde vereinfacht. Andererseits kann diese leicht gereinigt werden.

Nach der therapeutischen Energieapplikation kann die Entfaltung der Metallelektrode wieder rückgängig gemacht werden, wodurch eine Entfernung der Sonde aus dem Hohlraum leicht möglich ist.

Erfindungsgemäß ist die Außenkontur eines Armes etwa in dessen Mitte annähernd spiral-, zickzack-, oder mäanderförmig, wobei die Außenkontur an den beiden Endbereichen des Armes geradlinig ist. Hierdurch ist durch die Formgebung des Armes selbst ein Biegeverhalten vorgegeben, das die sternförmige Ausbildung der Metallelektrode begünstigt. Durch einen derartig ausgebildeten Mittelbereich eines Armes kann die Biegesteifigkeit den Anforderungen entsprechend angepasst werden.

Nach einer vorteilhaften Ausführungsform der Erfindung können die distalen und proximalen Enden der Arme mit Hilfe einer an einem proximalen Bereich des Schlauches vorgesehenen Betätigungseinrichtung relativ zueinander verstellbar sein. Hierdurch lässt sich der Außendurchmesser der Metallelektrode variieren und die räumliche Erstreckung von der zylindrischen Gestalt zu der sternförmigen Gestalt verändern.

Nach einer weiteren Ausführungsform der Erfindung sind die proximalen Enden der Arme mit einer flexiblen Kanüle verbunden, die innerhalb des Schlauches geführt ist. Diese Ausführungsform besitzt den Vorteil, dass innerhalb der flexiblen Kanüle Spülflüssigkeit und/oder ein weiteres Stellelement zur Bewegung der Arme geführt werden kann. Beispielsweise können die distalen Enden der Arme mit einem flexiblen Stellelement verbunden sein, das innerhalb des Schlauches bzw. innerhalb der Kanüle geführt ist. Auf diese Weise lässt sich der Außendurchmesser der Metallelektrode verändern, ohne dass dabei notwendigerweise der Schlauch mitbewegt wird.

Nach einer weiteren Ausführungsform der Erfindung kann im Bereich der distalen Enden der Arme ein Temperatursensor angeordnet sein. Eine solche Anordnung ermöglicht zusätzlich eine Temperaturkontrolle oder auch eine automatische Temperaturregelung über einen hierfür ausgebildeten Hochfrequenzgenerator.

Ferner kann es vorteilhaft sein, wenn ein proximales Ende der Sonde mit einem Anschluss für Spülflüssigkeit versehen ist. Diese Spülflüssigkeit kann durch den Schlauch bzw. die in dem Schlauch vorgesehene Kanüle in Richtung der Metallelektrode gefördert werden, um ein Austrocknen und einen elektrischen Leitfähigkeitsverlust in der Ablationszone zu vermeiden.

Eine Elektrode für eine Sonde der oben beschriebenen Art wird erfindungsgemäß dadurch hergestellt, dass ein flächiger Zuschnitt oder eine zylindrische Kanüle aus elektrisch leitendem Material mit parallel zueinander verlaufenden Mikroschnitten versehen wird, deren Enden sich jeweils nicht bis zum Rand des Zuschnitts oder der Kanüle erstrecken. Auf diese Weise werden zwischen den Mikroschnitten einzelne Stege gebildet, die als Arme der Sonde dienen. Hierbei werdendie Mikroschnitte in einem mittleren Abschnitt spiral-, mäander-, oder zickzackförmig ausgebildet. Hierdurch entstehen im mittleren Bereich der Arme biegeelastische Metallstege. Um die mittleren Abschnitte der Arme besonders flexibel auszubilden, kann es vorteilhaft sein, die Dicke der Mikroabschnitte in einem mittleren Abschnitt größer und in den Endabschnitten geringer zu wählen.

Nachfolgend wird die vorliegende Erfindung beispielhaft anhand einer vorteilhaften Ausführungsform und unter Bezugnahme auf die beigefügte Zeichnung beschrieben. Es zeigen:
- Fig. 1: eine schematische Ansicht einer Vorrichtung zur Hochfrequenz-Thermoablation;
- Fig. 2: eine teilweise geschnittene Ansicht eines distalen Endes einer Sonde, bei der die Metallelektrode eine zylindrische Gestalt aufweist; und
- Fig. 3: die Sonde von Fig. 2, wobei die Metallelektrode zu einer Sternform entfaltet ist.

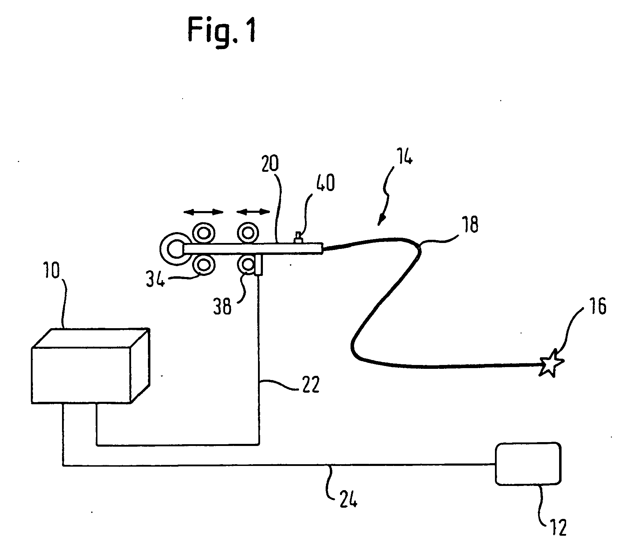

Fig. 1 zeigt schematisch ein System zur Hochfrequenz-Thermoablation mit einem Hochfrequenzgenerator 10, einer Neutralelektrode 12 und einer endoluminalen Sonde 14, an deren distalem Ende eine nur schematisch dargestellte Metallelektrode 16 vorgesehen ist. Die Metallelektrode 16 befindet sich am distalen Ende eines isolierenden Schlauches 18, an dessen proximalem Ende ein Handstück 20 vorgesehen ist. Das Handstück 20 weist einen Anschluss für ein Hochfrequenzkabel 22 auf, das den Hochfrequenzgenerator 10 elektrisch mit der Metallelektrode 16 verbindet. Die Neutralelektrode 12 ist über ein Neutralelektrodenkabel 24 mit dem Hochfrequenzgenerator 10 elektrisch verbunden.

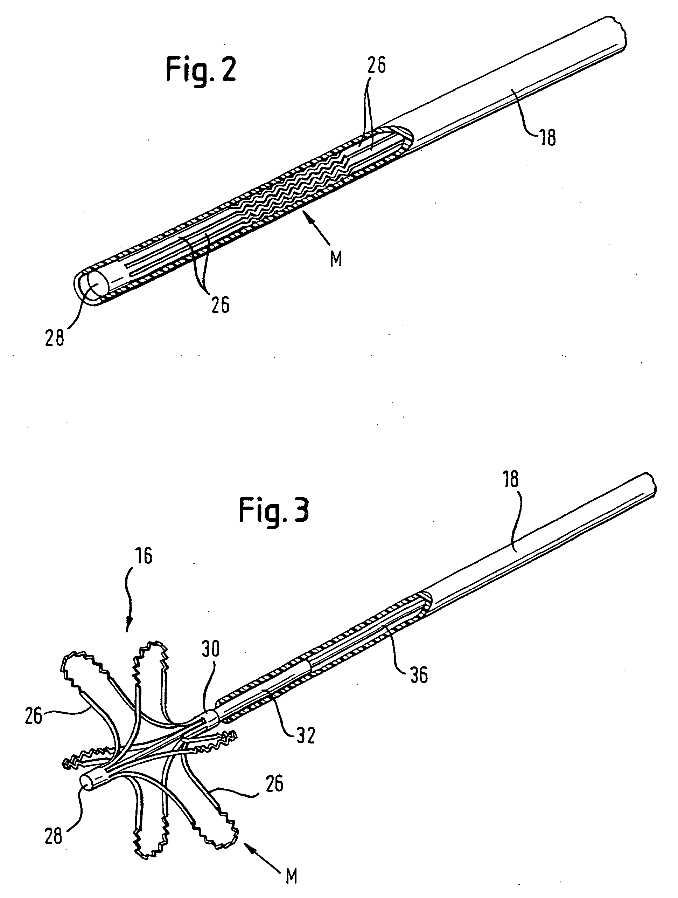

Fig. 2 zeigt in perspektivischer Ansicht den distalen Bereich der Sonde 14, wobei die Metallelektrode 16 zu einer zylindrischen Gestalt zusammengefaltet und in das Innere des Schlauches 18 zurückgezogen ist. Zur besseren Darstellung ist ein Teil des Schlauches 18 aufgebrochen gezeigt.

Fig. 3 zeigt die Sonde von Fig. 2, wobei jedoch die Metallelektrode 16 aus dem Schlauch 18 heraus geschoben ist und zu einer sternförmigen Gestalt entfaltet ist. Wie in den Figuren 2 und 3 zu erkennen ist, ist die Metallelektrode 16 durch beispielsweise sechs flexible Arme 26 gebildet, die in Fig. 2 koaxial zur Achse des Schlauches 18 verlaufen, die jedoch in Fig. 3 überwiegend radial und sternförmig von der Mittelachse der Sonde vorstehen. Sämtliche Arme 26 sind an ihren distalen Enden über ein topfartiges Endstück 28 miteinander verbunden. Die distalen Enden der Arme 26 sind über ein hülsenförmiges Endstück 30 ebenfalls miteinander verbunden. Die Arme 26 sowie die Endstücke 28 und 30, die sämtlich einstückig miteinander verbunden sind, bilden somit die Metallelektrode 16, die aufgrund ihrer äußerst kompakten Bauweise durch den Instrumentierkanal eines Endoskops geführt werden kann.

Wie insbesondere in den Fig. 2 und 3 zu erkennen ist, besteht jeder Arm 26 aus einem dünnen Metallstreifen mit rechteckigem Querschnitt, wobei die Form jedes Armes 26 etwa in dessen Mitte M von der Form an den beiden Enden des Armes abweicht. Die Außenkontur jedes Armes 26 ist etwa in dessen Mitte M zickzack-, spiral- oder mäanderförmig ausgebildet, während sich an diesen Bereich in der Mitte M zu beiden Seiten ein Abschnitt mit geradliniger Außenkontur anschließt. Hierbei weist der Abschnitt mit zickzack-, spiral- oder mäanderförmiger Außenkontur etwa ein Drittel der Gesamtlänge des Armes 26 auf.

Durch die zickzack-, spiral- oder mäanderförmige Außenkontur der Arme 26 in deren mittleren Bereich M lassen sich die Arme zu der in Fig. 3 dargestellten Anordnung biegen, bei der der zickzack-, spiral- oder mäanderförmige Abschnitt U-förmig gekrümmt ist und die Arme etwa rechtwinklig von der Mittelachse der Sonde vorstehen. Demgegenüber besitzt die Metallelektrode 16 in dem in Fig. 2 dargestellten Zustand, in dem sie in das Innere des Schlauches zurückgezogen ist, eine zylindrische Gestalt, bei der die einzelnen Arme 26 parallel zueinander verlaufen und mit nur geringem Abstand aneinander anliegen.

Das proximale Endstück 30 der Metallelektrode 16 ist mit einer innerhalb des Schlauches geführten, flexiblen Kanüle 32 verbunden, die mit einem Schiebegriff 34 (Fig. 1) des Handstückes 20 in Verbindung steht. Das distale Endstück 28 der Metallelektrode 16 ist mit einem flexiblen Stellelement 36 verbunden, das innerhalb der Kanüle 32 geführt ist und dessen proximales Ende mit einem Schiebegriff 38 in Verbindung steht, der ebenfalls an dem Handstück 20 vorgesehen ist. Das Stellelement 36 und die Kanüle 32 sind innerhalb des Handstückes 20 mechanisch lösbar mit den Schiebegriffen 34 und 38 verbunden, wobei die Schiebegriffe mit einer Hand einzeln und relativ zueinander betätigt werden können. Das proximale Ende des Schlauches 18 ist mit dem Gehäuse des Handgriffes 20 lösbar verbunden. Innerhalb des Handgriffes, der nicht näher dargestellt ist, ist eine elektrisch leitende Verbindung zwischen der Kanüle 32 und dem Metallelektrodenkabel 22 vorgesehen.

Durch relatives Bewegen der Schiebegriffe 34 und 38 zueinander und relativ zu dem Handstück 20 kann die Metallelektrode 16 von der in Fig. 2 dargestellten Position, in der sie sich vollständig innerhalb des Schlauches 18 befindet, in die in Fig. 3 dargestellte Anordnung verschoben werden, bei der die Metallelektrode 16 aus dem distalen Ende des Schlauches 18 vorsteht und eine sternförmige Gestalt aufweist.

Im Bereich des Handstückes 20 kann ferner ein Anschluss 40 für Spülflüssigkeit vorgesehen sein, durch den beispielsweise physiologische Kochsalzlösung in den Bereich der Metallelektrode 16 gebracht werden kann.

Der Schlauch 18 der Sonde 14 besteht aus flexiblem, isolierendem Material. Die Metallelektrode 16 und die Kanüle 32 bestehen aus einem superelastischen Werkstoff, der elektrisch leitet, beispielsweise aus einer Nickel-Titan-Legierung. Das Stellelement 36 kann wahlweise drahtförmig oder kanülenförmig sein und aus flexiblem Metall oder Kunststoff bestehen. Der Handgriff 20 und die Schiebegriffe 34, 38 bestehen aus isolierendem Kunststoff.

Die Sonde 14 kann eine je nach Anwendung unterschiedliche Gesamtlänge bis etwa 200 mm und einen Sondendurchmesser bis etwa 3 mm aufweisen. Die Metallelektrode 16 kann eine Länge von etwa 20 bis 35 mm aufweisen. Der Außendurchmesser der Metallelektrode 16 kann beispielsweise 1,8 mm betragen. Von der in Fig. 2 dargestellten Konfiguration kann die Metallelektrode durch Stauchung zu der in Fig. 3 dargestellten, sternförmigen Anordnung umgewandelt werden, in der der Elektrodenstern eine axiale Länge von etwa 8 mm und einen Durchmesser von bis zu 30 mm aufweist.

Die Herstellung der erfindungsgemäßen Metallelektrode 16 kann beispielsweise dadurch erfolgen, dass ein flächiger Zuschnitt, beispielsweise eine dünne Metallfolie, oder aber eine zylindrische Kanüle, jeweils aus elektrisch leitendem Material mit parallel zueinander verlaufenden Mikroschnitten versehen werden, wobei sich die Schnitte an ihren Enden jeweils nicht bis zum Rand des Zuschnittes oder der Kanüle erstrecken. Werden diese Schnitte gleichmäßig über den Umfang der Kanüle bzw. über die Erstreckung des Zuschnittes verteilt, so entstehen zwischen den einzelnen Schnitten streifenartige Materialabschnitte, welche die Arme 26 bilden. Diese Mikroschnitte werden in einem mittleren Abschnitt spiral-, mäander-, oder zickzackförmig (vgl. Fig. 2 und 3) ausgebildet, wodurch die vorstehend beschriebenen mittleren Abschnitte der Arme gebildet werden können. Der Zuschnitt kann anschließend zu einer Hülse geformt werden.

Außerdem kann es vorteilhaft sein, die Dicke der Mikroschnitte entlang deren Längserstreckung zu variieren. Beispielsweise kann die Dicke der Mikroschnitte in dem mittleren Abschnitt M größer als im Bereich der Endabschnitte gewählt werden, wodurch eine zusätzliche Variation des Elastizitätsverhaltens der Arme möglich ist.

Nachfolgend wird die Arbeitsweise der oben beschriebenen Sonde am Beispiel der endoluminalen Radiofrequenz-Thermoablation beschrieben: Nachdem der zu behandelnde Patient mit einer adhäsiven und großflächigen Neutralelektrode 12 auf der Haut kontaktiert ist, was in möglichst kurzer Körperdistanz zu der Metallelektrode 16 erfolgen sollte, ist der Patient nach Anschluss des Neutralelektrodenkabels 24 an die Neutralelektrode 12 und an den Hochfrequenzgenerator 10 ein integraler Bestandteil des Hochfrequenzstromkreises.

Anschließend wird die erfindungsgemäße Sonde über den Instrumentierkanal eines flexiblen Endoskops - oder bei interventionellen Therapien unter Bildkontrolle (Sonographie, Computertomographie, Magnetresonanztomographie) über Körperhöhlen oder Gefäße zum Zielgebiet navigiert. Anschließend wird die Metallelektrode 16 durch Betätigung der Schiebegriffe 34 und 38 ausgefahren und entfaltet, so dass diese das zu behandelnde oder pathogene Biogewebe, beispielsweise innerhalb der Speiseröhre, kontaktiert. Anschließend wird ein Hochfrequenzstrom an dem HF-Generator für eine definierte Zeit oder bis zum Erreichen eines vorgegebenen elektrischen Impedanzniveaus oder Energieniveaus oder auch Temperaturniveaus aktiviert. Hierbei fließt der Hochfrequenzstrom innerhalb der Sonde 14 über die sternförmig entfaltete Metallelektrode 16 zum kontaktierten Biogewebe und über die Neutralelektrode 12 zurück zum HF-Generator 10. Mit dem Quadrat der HF-Stromstärke und proportional zur aktuellen Kontaktimpedanz Elektrode-Biogewebe sowie proportional zur Aktivierungszeit entstehen örtlich begrenzte Wärmewirkungen, wobei bei erreichten Temperaturen über 50°C, vorzugsweise im Bereich 60°C bis 100°C das Biogewebe thermisch denaturiert wird. Nach sekundenlangen Einwirkzeiten des HF-Stromes werden im eng begrenzten Kontaktgebiet der Elektroden Biogewebeschrumpfungen und - austrocknungen erzielt. Somit kann auch malignes Biogewebe (Primärtumore, Metastasen) thermisch zerstört werden oder es kann eine thermische Hämostase oder auch therapeutische Gewebeschrumpfung mit tolerierbarer Narbenbildung erzeugt werden.

### Bezugszeichenliste

- 10: HF-Generator
- 12: Neutralelektrode
- 14: Sonde
- 16: Metallelektrode
- 18: Schlauch
- 20: Handstück
- 22: Metallelektrodenkabel
- 24: Neutralelektrodenkabel
- 26: Arme
- 28: distales Endstück
- 30: proximales Endstück
- 32: Kanüle
- 34: Schiebegriff
- 36: Stellelement
- 38: Schiebegriff
- 40: Anschluss für Spülflüssigkeit

- M: Mittlerer Bereich

## Patentansprüche

1. Sonde (14) zur Hochfrequenz-Thermoablation mit einem isolierenden Schlauch (18), an dessen distalem Ende eine Metallelektrode (16) vorgesehen ist; deren räumliche Erstreckung von einer zylindrischen Gestalt zu einer sternförmigen Gestalt veränderbar ist, wobei die Metallelektrode (16) durch mehrere flexible Arme (26) gebildet ist, deren distale und proximale Enden miteinander verbunden sind, wobei die distalen und/oder die proximalen Enden der Arme (26) elektrisch mit einem Anschluss zur Zuführung eines Hochfrequenzstromes verbunden sind,
**dadurch gekennzeichnet, dass**
die Außenkontur eines Armes (26) etwa in dessen Mitte (11) annähernd spiral-, zickzack-, oder mäanderförmig und an seinen beiden Enden geradlinig ist.

2. Sonde nach Anspruch 1,
**gekennzeichnet durch** eine an einem proximalen Bereich des Schlauches (18) vorgesehene Betätigungseinrichtung (34, 38),
mit deren Hilfe die distalen und die proximalen Enden der Arme (26) relativ zueinander verstellbar sind.

3. Sonde nach Anspruch 1,
**gekennzeichnet durch** eine flexible Kanüle (32),
die mit den proximalen Enden der Arme (26) verbunden ist und innerhalb des Schlauches (18) geführt ist.

4. Sonde nach Anspruch 1, **gekennzeichnet durch** ein flexibles Stellelement (36), das mit den
distalen Enden der Arme (26) verbunden ist und innerhalb des Schlauches (18) geführt ist.

5. Sonde nach Anspruch 1,
**dadurch gekennzeichnet, dass**
im Bereich (28) der distalen Enden der Arme (26) ein Temperatursensor angeordnet ist.

6. Sonde nach Anspruch 1,
**dadurch gekennzeichnet, dass**
deren proximales Ende mit einem Anschluss (40) für Spülflüssigkeit versehen ist.

7. Verfahren zur Herstellung einer Elektrode für eine Sonde nach Anspruch 1, wobei
in einen flächigen Zuschnitt oder eine zylindrische Kanüle aus elektrisch leitendem Material parallel zueinander verlaufende Mikroschnitte eingebracht werden, die sich an ihren Enden jeweils nicht bis zum Rand des Zuschnittes oder der Kanüle erstrecken,
**dadurch gekennzeichnet, dass**
die Mikroschnitte in einem mittleren Abschnitt spiral-, mäander-, oder zickzackförmig ausgebildet werden.

8. Verfahren nach Anspruch 7,
**dadurch gekennzeichnet, dass**
die Dicke der Mikroschnitte in einem mittleren Abschnitt größer und in Endabschnitten geringer gewählt wird.

## Claims

1. A probe (14) for high-frequency thermal ablation, with an insulating tube (18), at the distal end of which there being provided a metal electrode (16), the spatial extension of which being variable from a cylindrical form to a star-shaped form, wherein the metal electrode (16) is formed by a plurality of flexible arms (26), the distal and proximal ends of which being connected to each other, wherein the distal and/or the proximal ends of the arms (26) are electrically connected to a terminal for supplying a high-frequency current,
**characterized in that**
the outer contour of an arm (26) is almost spiral, zigzag, or meander-shaped approximately in the centre (11) thereof, and rectilinear at the two ends thereof.

2. The probe according to claim 1,
**characterized by**
an operation device (34, 38) provided at a proximal area of the tube (18), with the help of which the distal and the proximal ends of the arms (26) are adjustable relative to each other.

3. The probe according to claim 1,
**characterized by**
a flexible cannula (32), which is connected to the proximal ends of the arms (26) and guided within the tube (18).

4. The probe according to claim 1,
**characterized by**
a flexible actuator (36), which is connected to the distal ends of the arms (26) and guided within the tube (18).

5. The probe according to claim 1,
**characterized in that**
a temperature sensor is arranged in the area (28) of the distal ends of the arms (26).

6. The probe according to claim 1,
**characterized in that**
the proximal end thereof is provided with a connection (40) for flushing liquid.

7. A method for producing an electrode for a probe according to claim 1, wherein mutually parallel microcuts are made in a flat cut section or a cylindrical cannula of electrically conductive material, which microcuts do at their ends each not extend up to the edge of the cut section or the cannula,
**characterized in that**
in a middle portion the microcuts are formed in a spiral, zigzag, or meander shape.

8. The method according to claim 7,
**characterized in that**
the thickness of the microcuts in a middle portion is selected to be greater, and smaller in end portions.

## Revendications

1. Sonde (14) pour ablation thermique à haute fréquence avec un tuyau isolant (18), à l'extrémité distale de laquelle on prévoit une électrode métallique (16), l'étendue spatiale de laquelle étant transformable d'une forme cylindrique à une forme d'étoile, sachant que l'électrode métallique (16) est constituée de plusieurs bras flexibles (26), dont les extrémités distales et proximales sont raccordées entre elles, sachant que les extrémités distales et/ou les extrémités proximales des bras (26) sont connectées électriquement à une prise d'alimentation en courant à haute fréquence,
**caractérisée en ce que**
le contour extérieur d'un bras (26) présente à peu près au centre (11) de celui-ci une forme approximativement en spirale, en zigzag, ou en méandres et il est rectiligne à ses deux extrémités.

2. Sonde d'après la revendication 1, **caractérisée par** un dispositif d'actionnement (34, 38) prévu dans une zone proximale du tuyau (18), à l'aide duquel les extrémités distales et proximales des bras (26) sont ajustables l'une par rapport à l'autre.

3. Sonde d'après la revendication 1, **caractérisée par** une canule flexible (32), qui est raccordée aux extrémités proximales des bras (26) et qui est guidée à l'intérieur du tuyau (18).

4. Sonde d'après la revendication 1, **caractérisée par** un élément de réglage (36) flexible, qui est raccordé aux extrémités distales des bras (26) et qui est guidé à l'intérieur du tuyau (18).

5. Sonde d'après la revendication 1, **caractérisée en ce que** dans la zone (28) des extrémités distales des bras (26) est disposé un capteur de température.

6. Sonde d'après la revendication 1, **caractérisée en ce que** son extrémité proximale est munie d'un branchement (40) pour un liquide de lavage.

7. Procédé de fabrication d'une électrode pour une sonde d'après la revendication 1, sachant que
dans une pièce découpée plane ou dans une canule cylindrique en un matériau électriquement conducteur on produit des coupes microscopique qui s'étendent parallèlement l'une par rapport à l'autre et qui à leurs extrémités ne s'étendent respectivement pas jusqu'au bord de la pièce découpée ou de la canule,
**caractérisé en ce que**
dans une section centrale les coupes microscopiques sont réalisées de manière à présenter une forme en spirale, en méandres, ou en zigzag.

8. Procédé d'après la revendication 7,
**caractérisé en ce que**
l'épaisseur des coupes microscopiques est choisie de manière à ce qu'elle soit plus grande dans une section centrale et plus petite dans des sections terminales.
